(19) Europäisches Patentamt
European Patent Office
Office européen des brevets

(11) Veröffentlichungsnummer: **0 315 912 B1**

(12) **EUROPÄISCHE PATENTSCHRIFT**

(45) Veröffentlichungstag der Patentschrift: **17.06.92**

(51) Int. Cl.$^5$: **A61K 7/06**

(21) Anmeldenummer: **88118408.9**

(22) Anmeldetag: **04.11.88**

(54) **Sebosuppressive Zubereitungen.**

(30) Priorität: **12.11.87 DE 3738405**

(43) Veröffentlichungstag der Anmeldung:
**17.05.89 Patentblatt 89/20**

(45) Bekanntmachung des Hinweises auf die Patenterteilung:
**17.06.92 Patentblatt 92/25**

(84) Benannte Vertragsstaaten:
**AT BE CH DE ES FR GB GR IT LI LU NL SE**

(56) Entgegenhaltungen:
**EP-A- 0 140 033**
**EP-A- 0 191 285**
**WO-A-83/02390**
**DE-A- 1 467 955**

(73) Patentinhaber: **Henkel Kommanditgesellschaft auf Aktien**
**Postfach 1100 Henkelstrasse 67**
**W-4000 Düsseldorf 1(DE)**

(72) Erfinder: **Möller, Hinrich, Dr.**
**Haydnstrasse 27**
**W-4019 Monheim(DE)**
Erfinder: **Wallat, Siegfried, Dr.**
**Marie-Curie-Strasse 9**
**W-4019 Monheim(DE)**

EP 0 315 912 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind topisch anzuwendende Zubereitungen zur Bekämpfung der übermäßigen Entwicklung von Hautfett und der dadurch hervorgerufenen Erkrankungen des seborrhoischen Formenkreises und zur Verringerung des fettigen, unästhetischen Aussehens der Haare und der Haut. Diese enthalten eine Kombination aus einem antiseborrhoisch wirksamem Alkyl-, Alkenyl- oder Alkylbenzyl-Arylether-Derivat und einer synergistisch wirksamen Verbindung, ausgewählt aus Antioxidantien, antimikrobiellen Wirkstoffen und aliphatischen $C_8$-$C_{20}$-Alkoholen.

Übermäßige Absonderungen der Talgdrüsen der Oberhaut können zu krankhaften Hautzuständen führen. In häufiger vorkommenden, leichteren Fällen stellen sie ein kosmetisches Problem dar, das sich durch fettiges Aussehen der Haare oder ein glänzend öliges Aussehen der Haut manifestiert. Die moderne Kosmetik ist daher bemüht, durch geeignete topisch anwendbare Zubereitungen die Sekretion der Talgdrüsen zu normalisieren und dem Haar und der Haut wieder ein ansprechendes Aussehen zu verleihen.

Obwohl bereits eine große Zahl von antiseborrhoisch wirksamen synthetischen Produkten vorgeschlagen wurde, besteht weiterhin ein Bedürfnis an Zubereitungen mit einer erhöhten Wirksamkeit bei niedrigen Anwendungskonzentrationen. Aufgabe der Erfindung ist es, antiseborrhoische Zubereitungen bereitzustellen, die gegenüber bekannten Präparaten eine verstärkte Wirkung, ohne nachteilige Nebenwirkungen auf den menschlichen Körper, haben.

4-Alkoxy-benzylalkohole sind bereits in der deutschen Patentanmeldung DE-A 33 32 505, 4-Alkoxy-benzoesäureester und 4-Alkylbenzyloxybenzoesäureester in den deutschen Patentanmeldungen DE-A 31 21 064, DE-A 33 01 313 und DE-A 35 00 971 und 4-Alkoxy-benzoesäuren und deren Salze in den deutschen Patentanmeldungen DE-A 30 47 106 und DE-A 35 00 972 als antiseborrhoische Zusätze für kosmetische Zubereitungen vorgeschlagen worden.

Auch Antioxidantien und langkettige Alkohole mit 12 bis 26 C-Atomen sind bereits als antiseborrhoische Zusätze für kosmetische Zubereitungen aus DE-A 32 01 511 bekannt.

Es wurde nunmehr gefunden, daß sich die antiseborrhoische Wirkung der genannten Produkte durch Kombination in synergistischer Weise steigern läßt, d. h., daß die genannten Verbindungen in einer Konzentration, in der sie allein völlig oder nahezu völlig unwirksam sind, in Kombination miteinander sehr gute antiseborrhoische Wirkungen entfalten. Darüber hinaus wurde festgestellt, daß auch bestimmte antimikrobielle Stoffe, die als Antischuppenwirkstoffe bekannt sind, die antiseborrhoischen Wirkungen der 4-Alkoxy-benzylalkohole und 4-Alkoxy-benzoesäuren und deren Ester und Salze in synergistischer Weise steigern. Dadurch ergibt sich die Möglichkeit, sebosuppressive Zubereitungen mit sehr niedrigen Einsatzkonzentrationen - und damit stark verringertem Nebenwirkungs-Risiko - herzustellen, die sich auch für den Einsatz in Kosmetika eignen.

Gegenstand der Erfindung sind sebosuppressive Zubereitungen, enthaltend

(a) eine oder mehrere antiseborrhoisch wirksame Verbindung(en) der Formel (I)

(I) 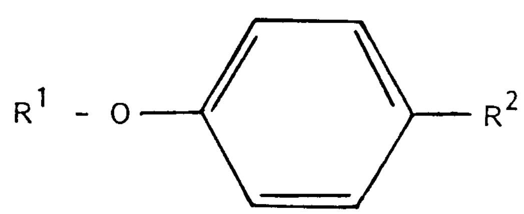

in der $R^1$ eine lineare oder verzweigte, gesättigte oder ungesättigte, aliphatische oder cycloaliphatische Alkylgruppe mit bis zu 20 C-Atomen, eine Hydroxyalkylgruppe mit 2 bis 20 C-Atomen, eine Alkoxyalkylgruppe mit insgesamt 3 bis 20 C-Atomen oder eine Benzylgruppe ist, die am Ring durch ein Halogen, eine Alkyl- oder Alkoxygruppe mit 1 bis 6 C-Atomen substituiert sein kann, und $R^2$ eine Hydroxyalkylgruppe mit 1 bis 4 C-Atomen, eine Gruppe $-COOR^3$, $-CH_2-COOR^3$, $-CH_2-CH_2-COOR^3$ oder $-CH=CH-COOR^3$ ist, worin $R^3$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy- und 2 bis 4 C-Atomen in der Alkylgruppe oder Wasserstoff oder ein salzbildendes Kation ist und

(b) eine oder mehrere synergistisch wirkende Verbindungen, ausgewählt aus

(b1) Antioxidantien, bevorzugt aus der Gruppe der Tocopherole, der tert. butylsubstituierten Phenole und der Gallussäureester

(b2) antimikrobiellen Wirkstoffen, bevorzugt aus der Gruppe

1-Hydroxy-2-pyridone und deren Salze
1-Hydroxy-2-pyridin-thione und deren Salze
Bis-(2-pyridyl-1-oxid)-disulfid und deren Erdalkalisalz-addukte und
1-(4-Chlorphenoxy)-1-(1-imidazolyl)-3,3-dimethyl-2-butanon und
(b3) aliphatischen, verzweigten und einfach oder mehrfach ungesättigten Terpenalkoholen mit 8 bis 26 C-Atomen.

Die antiseborrhoisch wirksamen Verbindungen der Formel (I) sind weitgehend literaturbekannt, z. B. aus den genannten Patentanmeldungen DE-A 33 32 505, DE-A 31 21 064, DE-A 33 01 313, DE-A 35 00 971, DE-A 30 47 106 und DE-A 35 00 972. Soweit Verbindungen aus den genannten Druckschriften nicht bekannt sind, lassen sie sich nach den in diesen Druckschriften angegebenen Verfahren synthetisieren. Die p-Alkoxy-benzoesäure-methylester werden z. B. durch Alkylierung von p-Hydroxybenzoesäure-methylester mit den Halogeniden der Formel $R^1$-X, worin X z. B. Chlor oder Brom ist, oder mit entsprechenden Sulfaten hergestellt. Die Hydroxyalkyl- und Alkoxyalkylester können dann aus den entsprechenden p-Alkoxybenzoesäure-methylestern durch Umesterung mit der jeweiligen Alkoholkomponente $R^3$-OH in Gegenwart alkalischer Katalysatoren, wie z. B. Natriumalkoholaten, hergestellt werden. Man kann auch umgekehrt zunächst die Veresterung der p-Hydroxybenzoesäure und danach die Alkylierung durchführen.

Analog zu den Alkoxybenzoesäure-Estern lassen sich auch die Alkoxyzimtsäureester, die p-Alkoxyphenyl-propionsäureester und die p-Alkoxyphenyl-ethansäureester aus den entsprechenden p-Hydroxyphenyl-carbonsäuremethylestern herstellen. Die freien Säuren, in welchen $R^3$ = Wasserstoff ist, lassen sich aus den entsprechenden Methylestern leicht durch Verseifung (Hydrolyse) gewinnen. Durch Neutralisation mit Basen lassen sie sich in die Salze überführen, in welchen $R^3$ das salzbildende Kation ist. Besonders gut geeignete, dermatologisch verträgliche Salze sind die Alkali- und Erdalkalimetallsalze, z. B. die Natrium-, Kalium-, Calcium- oder Magnesiumsalze, aber auch die Ammonium- und Alkanolammoniumsalze, z. B. das Monoethanolammoniumsalz, das Isopropanolammoniumsalz oder das Triethanolammoniumsalz. Es sind aber auch Salze anderer Basen wirksam, soweit diese ausreichend dermatologisch verträglich sind.

Die Verbindungen der allgemeinen Formel (I), in welchen $R^2$ eine Hydroxyalkylgruppe ist, lassen sich nach literaturbekannten Verfahren aus den entsprechenden Carbonsäuremethylestern durch Reduktion mit komplexen Metallhydriden, z. B. mit Natriumborhydrid, Lithium-aluminium-hydrid oder mit Natrium-bis-(2-methoxyethoxy)-aluminiumhydrid (Vitride$^{(R)}$) herstellen.

Als synergistisch wirkende, die antiseborrhoische Wirkung der Verbindungen der Formel (I) verstärkende Komponente (b) aus der Gruppe der Antioxidantien (b1) sind bevorzugt die Tocopherole und die tert. butylsubstituierten Phenole geeignet. Von den Tocopherolen zeigen alle vier Homologen eine synergistische Wirkung, also das alpha-Tocopherol(5,7,8-Trimethyl-tocol), das beta-Tocopherol(5,8-Dimethyl-tocol), das gamma-Tocopherol(7,8-Dimethyltocol) und das delta-Tocopherol(8-Monomethyl-tocol) und die optischen Isomeren sowie die Razemate und die Acetate der genannten Tocopherole. Auch Tocopherolgemische, wie sie z. B. aus pflanzlichen Samenölen isoliert werden und Tocotienole sind synergistisch wirksam. Von den tert. butylsubstituierten Phenolen eignen sich bevorzugt das 2,6-Di-tert. butyl-4-methylphenol, die isomeren 2- bzw. 3-tert.butyl-4-methoxyphenole sowie das tert. Butylhydrochinon.

Von den antimikrobiellen Wirkstoffen (b2) eignen sich besonders solche, die eine fungizide Wirkung gegen Pityrosporum ovale, den Sproßpilz, der als eine der Hauptursachen für die Bildung von Kopfschuppen gilt, aufweisen und deshalb als Wirkstoffe gegen Kopfschuppen geeignet sind. Bevorzugt eignen sich hierfür vor allem Verbindungen vom Typ der 1-Hydroxy-2-pyridone der allgemeinen Formel (II)

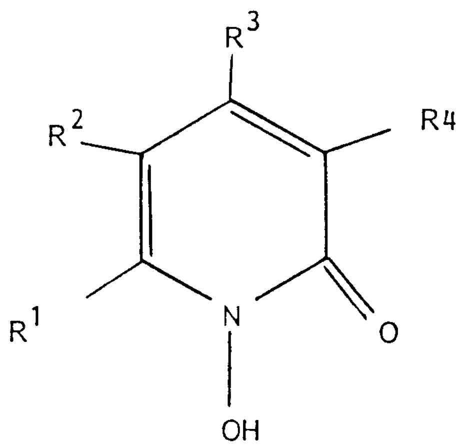

(II)

worin $R^1$ für Wasserstoff, Alkyl mit 1 bis 17 Kohlenstoffatomen, Alkenyl mit 2 bis 17 Kohlenstoffatomen, Cycloalkyl mit 5 bis 8 Kohlenstoffatomen, Cycloalkyl-alkyl mit Alkyl von 1 bis 4 Kohlenstoffatomen, wobei die Cycloalkylreste durch Alkylgruppen mit 1 bis 4 Kohlenstoffatomen substituiert sein können, Aryl-, Aralkyl mit Alkyl von 1 bis 4 Kohlenstoffatomen, Arylalkenyl mit Alkenyl von 2 bis 4 Kohlenstoffatomen, Aryloxyalkyl oder Arylmercaptoalkyl mit Alkyl von 1 bis 4 Kohlenstoffatomen, Benzhydryl, Phenylsulfonylalkyl mit Alkyl von 1 bis 4 Kohlenstoffatomen, Furyl oder Furylalkenyl mit Alkenyl von 2 bis 4 Kohlenstoffatomen steht und worin die vorgenannten Arylreste durch Alkylgruppen mit 1 bis 4 Kohlenstoffatomen, Alkoxygruppen mit 1 bis 4 Kohlenstoffatomen, Nitrogruppen, Cyangruppen oder Halogen substituiert sein können, $R^2$ Wasserstoff, Alkyl mit 1 bis 4 Kohlenstoffatomen, Alkenyl- oder Alkinyl mit 2 bis 4 Kohlenstoffatomen, Halogen oder den Benzylrest bedeutet, $R^3$ für Wasserstoff, Alkyl mit 1 bis 4 Kohlenstoffatomen oder Phenyl steht und $R^4$ Wasserstoff, Alkyl mit 1 bis 4 Kohlenstoffatomen, Alkenyl mit 2 bis 4 Kohlenstoffatomen, Methoxymethyl, Halogen oder ein Benzylrest sein kann und/oder deren Salze.

Die Verwendung dieser Verbindungen als Antischuppen-Wirkstoffe ist aus DE-A 22 34 099 bekannt. Von den Verbindungen dieser Gruppe kommt dem 1-Hydroxy-4-methyl-6(2,4,4-trimethylpentyl)-2-pyridon, dessen Monoethanolamin-Salz als OCTOPIROX(R) oder PIROCTONE-OLAMINE im Handel erhältlich ist, besondere Bedeutung zu.

Weitere geeignete antimikrobielle Stoffe mit Antischuppen-Wirkung sind das aus DE-A 12 01 508 bekannte 1-Hydroxy-2-pyridinthion und dessen Salze, bevorzugt dessen Zinksalz, das aus DE-A 22 48 880 bekannte Bis-(2-pyridyl-1-oxid)-disulfid und dessen Addukte an Erdalkalimetallsalze, insbesondere die Addukte an Magnesiumsalze. Schließlich eignet sich auch das aus DE-A 24 30 039 bekannte 1-(4-Chlorphenoxy)-1-(1-imidazolyl)-3,3-dimethyl-2-butanon, welches unter der Handelsbezeichnung CLIMBAZOL(R) erhältlich ist, als synergistisch wirksame Komponente (b).

Von den synergistisch wirkenden Alkoholen der Gruppe (b3) eignen sich vor allem die, welche verzweigt und/oder einfach oder mehrfach ungesättigt sind und 8 bis 26 C-Atome enthalten. Geeignete verzweigte Alkohole sind z. B. Isononylalkohol, Isotridecylalkohol, 2-Hexyl-decanol, 2-Octyl-dodecanol, Isooctadecylalkohol, 2-(1,3,3-Trimethylbutyl)-5,7,7-trimethyloctanol, 2-Methyl-undecanol, 2-Methyl-tetradecanol, Oleylalkohol, Linoleylalkohol. Bevorzugt eignen sich verzweigte, einfach oder mehrfach ungesättigte Terpenalkohole wie z. B. Farnesol, Phytol, Geraniol, Nerol, Nerolidol und Isophytol.

Besonders gut ausgeprägt ist der Synergismus zwischen (a) der antiseborrhoischen Verbindung der Formel (I) und der synergistisch wirkenden Verbindung (b) in Zusammensetzungen, in welchen in der antiseborrhoisch wirksamen Verbindung der Formel (I) $R^1$ eine verzweigte, eine cycloaliphatische und/oder eine einfach oder mehrfach ungesättigte Alkylgruppe mit 8 bis 20 C-Atomen und $R^2$ eine Gruppe $-CH_2OH$, $-COOR^3$, $-CH_2-COOR^3$, $CH_2-CH_2-COOR^3$ oder $-CH=CH-COOR^3$ ist, worin $R^3$ eine Alkylgruppe mit 1 bis 4 C-Atomen, Wasserstoff oder ein salzbildendes Kation ist.

Besonders bevorzugt ist $R^1$ eine Isononyl-, Isotridecyl-, 3-Cyclohexyl-propyl-, 4-(1,3,3-Trimethyl-2-cyclohexyl)-2-butyl-cyclohexylmethyl-, Phytyl-, Farnesyl- oder Geranyl-Gruppe.

Eine weitere bevorzugte Ausführungsform sind erfindungsgemäße Zubereitungen, in welchen die synergistisch wirksame Verbindung (b) ausgewählt ist aus

(b1) D-alpha-Tocopherol, D,L-alpha-Tocopherol, Tocopherolgemischen und 2,6-Di-tert.butyl-4-methylphenol

(b2) 1-Hydroxy-4-methyl-6-(2,4,4-trimethylpentyl)-2-pyridon oder dessen Salzen

(b3) Phytol und Farnesol.

Die antiseborrhoisch wirksamen Verbindungen der Formel (I) werden bevorzugt in einer Menge von 0,0001 bis 0,5 Gew.-% in die erfindungsgemäßen Zubereitungen eingesetzt. Durch die Kombination mit der synergistischen Komponente (b) wird aber in vielen Fällen bereits bei 0,0001 bis 0,1 Gew.-% der Verbindungen der Formel (I) eine gute Wirkung erzielt. Die synergistisch wirksamen Verbindungen (b), die allein auch bei höheren Konzentrationen keine nennenswerten antiseborrhoischen Wirkungen aufweisen, werden in die erfindungsgemäßen Zubereitungen in einer Menge von 0,001 bis 1,0 Gew.-%, bevorzugt von 0,001 bis 0,1 Gew.-%, bezogen auf die gesamte Zusammensetzung, eingesetzt. Das Gewichtsverhältnis von antiseborrhoisch wirksamen Verbindungen (a) der Formel (I) zu Synergisten (b) beträgt in den erfindungsgemäßen Zubereitungen bevorzugt 1 : 5 bis 1 : 50.

Die erfindungsgemäßen Kombinationen aus einer antiseborrhoisch wirksamen (a) Verbindung der Formel (I) und einer erfindungsgemäßen synergistischen Komponente (b) besitzen eine ausgeprägte sebosuppressive Wirkung bei außerordentlich niedrigen Anwendungskonzentrationen der Einzelkomponenten. Sie sind darüber hinaus ausgezeichnet haut- und schleimhautverträglich und lassen sich problemlos in die unterschiedlichsten pharmazeutischen und kosmetischen Träger einarbeiten.

Als kosmetische Träger eignen sich alle für die Aufbringung auf die Haare oder die Haut geeigneten Zubereitungen. Für die Hautbehandlung eignen sich insbesondere wäßrige oder alkoholische Lösungen, tensidhaltige Lotionen, Öle, Salben, Emulsionen, Cremes, Gele und Stiftpräparate. Für die Haarbehandlung eignen sich besonders Haarwässer, Haarshampoos, Haarkuren, Haarspülungen und Haarsprays. Wegen der besonderen kosmetischen Probleme, die durch fettendes Haar verursacht werden, stellen die haarkosmetischen Zubereitungen besonders bevorzugte Ausführungsformen der Erfindung dar.

Die wichtigsten Komponenten üblicher kosmetischer Träger sind

- Ölkomponenten, z. B. Paraffinöl, Pflanzenöle, Fettsäureester, Squalan, Fettalkohole, 2-Octyldodecanol,
- Fette und Wachse, z. B. Walrat, Bienenwachs, Montanwachs, Paraffin, Cetyl-stearylalkohol,
- Emulgatoren, z. B. Fettsäurepartialglyceride, Fettsäure-Sorbitan-partialester und deren Ethoxylate, Seifen, Fettalkoholsulfate, Fettalkoholpolyglycolether, Alkylphosphate,
- Waschrohstoffe, insbesondere Aniontenside, z. B. Fettalkoholpolyglycolethersulfate, Fettalkoholsulfate, Alphaolefinsulfonate, Alkansulfonate, Sulfobernsteinsäureester, Acyltauride, Acylisethionate und Acylsarkosine,

  ampholytische Tenside, z. B. N-Alkylglycin, N-Alkylaminopropionsäure, N-Alkylaminobuttersäure mit 8 bis 18 C-Atomen in der Alkylgruppe, zwitterionische Tenside, z. B. N-Alkyl($C_8$-$C_{18}$)- N,N-dimethylammonio-glycinat oder

  N-Kokosacylaminopropyl-N,N-dimethylammonioglycinat und

  nichtionogene Tenside, z. B. Fettalkoholpolyglycolether, Alkylphenolpolyglycolether, Fettsäurepolyglycolester, Aminoxid-Tenside, Fettsäurealkanolamide und deren Ethoxylate und

  kationische Tenside, z. B. Alkyl($C_{12}$-$C_{18}$)trimethylammoniumchlorid, Lauryldimethylbenzylammoniumchlorid, Cetylpyridiniumchlorid, Distearyldimethylammoniumchlorid
- niedere Alkohole wie z. B. Ethanol, Isopropanol,
- mehrwertige Alkohole wie z. B. Ethylenglycol, Propylenglycol, Glycerin,
- Wasser und Hilfsstoffe wie z. B. Duftstoffe, Konservierungsmittel, Puffersubstanzen, Verdickungsmittel, Farbstoffe und Trübungsmittel.

Die folgenden Beispiele sollen den Erfindungsgegenstand näher erläutern:

## Beispiele

### 1. Prüfung und Bewertung der antiseborrhoischen Wirkung Grundlage

Der Test geht von der Beobachtung aus, daß männliche Ratten ein bräunliches Hautfett absondern, so daß die mehr oder weniger starke Fettigkeit der Haut visuell gut als Hautbräunung beurteilt werden kann. Daß es sich bei der Bräunung um Hautoberflächenfett handelt, ist daran zu erkennen, daß junge weibliche Ratten sowie männliche Ratten nach dem Waschen mit Tensidlösungen oder mit Lipidlösungsmitteln oder auch männliche Ratten, die systemisch mit Östrogen behandelt wurden, nur die normale helle, rosafarbene Haut nach dem Scheren aufweisen. Parallel dazu sind aus den abgeschnittenen Haaren nur noch sehr geringe Lipidmengen zu extrahieren (vgl. hierzu auch J. Soc. Cosmet. Chem. 34, 127 bis 135 (1983)).

### Durchführung

Als Versuchstiere dienten männliche Wistar-Ratten mit einem Körpergewicht von 220 bis 230 g zu Versuchsbeginn.

Zur Beurteilung der Wirksamkeit wurden die Prüfsubstanzen in den in Tabelle 1 angegebenen Konzentrationen in Ethanol/Aceton (1 : 1) gelöst und jeweils 6 Ratten halbseitig auf das Rückenfell gepinselt. Die andere Seite wurde nur mit dem Lösungsmittel behandelt.

Während der Versuchsdauer von 14 Tagen wurde an insgesamt 9 Tagen einmal appliziert. Zur weiteren Kontrolle diente eine Gruppe von 6 Ratten, die beidseitig nur mit dem Lösungsmittel behandelt wurden. Am Ende des Versuchs wurden die Tiere am Rücken und an den Flanken geschoren und von einem Beurteilerpanel (6 Personen) unabhängig unter Doppelblindbedingungen visuell abgemustert. Dabei wurde der Bräunungsgrad auf dem Rücken der Ratten als Maß für den Hautfettbelag visuell beurteilt.

Bewertung

Als 1. Kriterium wurde der Unterschied zwischen rechter und linker Seite gewertet, wobei pro Beurteiler und Tier jeweils 1 Punkt zu vergeben war, und zwar in der Weise, daß die

| | |
|---|---|
| dunklere Seite | mit 1 Punkt |
| hellere Seite | mit 0 Punkten und |
| bei Gleichheit beide Seiten | mit 0,5 Punkten |

benotet wurden.

Signifikante Differenzen zwischen nur mit dem Lösungsmittel behandelter und mit Prüflösung behandelter Seite nach dieser Bewertungsmethode zeigen die lokale Wirksamkeit einer Substanz an.

Als 2. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach dieser Bewertungsmethode werden die Punktsummendifferenzen zwischen den unbehandelten Kontrolltieren und jeweils den behandelten und unbehandelten Seiten ($\Delta P$) der Versuchstiere gebildet, wobei wiederum signifikante Differenzen zwischen Kontrolltieren und der behandelten Seite der Versuchstiere die Wirkung einer Substanz deutlich machen.

Prozentuale Sebumreduktion

Die Sebumreduktion errechnet sich aus der Punktedifferenz in der Weise, daß man den Quotienten aus der Punktedifferenz $\Delta P$ und der Punktezahl für die Kontrollgruppe $P_k$ bildet und den erhaltenen Wert in % angibt.

$$\text{Sebumreduktion} = \frac{\Delta P}{P_k} \cdot 100 \quad (\%)$$

Tabelle I

| | Konz. | Prozentuale Sebumreduktion |
|---|---|---|
| Decyloxybenzylalkohol | 0,01 | 0 |
| Dodecyloxybenzylalkohol | 0,01 | 0 |
| Isononyloxybenzylalkohol<br>Isononyloxybenzylalkohol | 0,005<br>0,002 | 31<br>0 |
| Isononyloxybenzoesäure<br>Isononyloxybenzoesäure | 0,02<br>0,01 | 27<br>0 |
| Isotridecyloxybenzoesäure<br>Isotridecyloxybenzoesäure | 0,002<br>0,001 | 10<br>0 |
| Cyclohexylpropoxy-benzoesäure | 0,005 | 0 |
| D-alpha-Tocopherol | 0,1 | 0 |
| D,L-alpha-Tocopherol | 0,05 | 0 |
| 2,6-Di-tert.butyl-4-methylphenol | 0,1 | 0 |
| Octopirox(R)<br>Octopirox(R) | 0,1<br>0,05 | 46<br>0 |
| Phytol<br>Phytol | 0,05<br>0,02 | 26<br>0 |
| Farnesol | 0,1 | 0 |

Tabelle II

| Beispiele | 1 | 2 | 3 | 4 | 5 | 6 | 7 | 8 | 9 | 10 |
|---|---|---|---|---|---|---|---|---|---|---|
| Decyloxybenzylalkohol | - | - | 0,01 | - | - | - | - | - | - | - |
| Dodecyloxybenzylalkohol | - | - | - | - | - | - | - | - | - | - |
| Isononyloxybenzylalkohol | - | 0,005 | - | - | - | - | 0,005 | 0,002 | - | - |
| Isononyloxybenzoesäure | - | - | - | - | - | - | - | - | 0,02 | 0,01 |
| Isotridecyloxybenzoesäure | - | - | - | 0,002 | 0,002 | 0,001 | - | - | - | - |
| Cyclohexylpropoxybenzoesäure | 0,005 | - | - | - | - | - | - | - | - | - |
| D-alpha-Tocopherol | 0,1 | - | - | 0,02 | 0,02 | 0,1 | - | - | - | - |
| D,L-alpha-Tocopherol | - | 0,05 | - | - | - | - | - | - | - | - |
| 2,6-Di-tert.butyl-4-methylphenol | - | - | 0,1 | - | - | - | - | - | - | - |
| Octopirox(R) | - | - | - | - | - | - | 0,1 | 0,05 | 0,1 | 0,05 |
| Phytol | - | - | - | - | - | - | - | - | - | - |
| Farnesol | - | - | - | - | - | - | - | - | - | - |
| Prozentuale Sebumreduktion | 32 | 41 | 26 | 45 | 29 | 26 | 75 | 46 | 89 | 59 |

Tabelle II (Fortsetzung)

| Beispiele | 11 | 12 | 13 | 14 | 15 | 16 | 17 | 18 |
|---|---|---|---|---|---|---|---|---|
| Decyloxybenzylalkohol | - | - | - | - | - | - | - | - |
| Dodecyloxybenzylalkohol | - | 0,01 | - | - | - | - | - | - |
| Isononyloxybenzylalkohol | - | - | 0,002 | - | - | - | - | - |
| Isononyloxybenzoesäure | - | - | - | - | - | - | - | - |
| Isotridecyloxybenzoesäure | 0,002 | - | - | 0,002 | 0,002 | 0,001 | 0,001 | 0,0005 |
| Cyclohexylpropoxybenzoesäure | - | - | - | - | - | - | - | - |
| D-alpha-Tocopherol | - | - | - | - | - | - | - | - |
| D,L-alpha-Tocopherol | - | - | - | - | - | - | - | - |
| 2,6-Di-tert.butyl-4-methylphenol | - | - | - | - | - | - | - | - |
| Octopirox(R) | 0,05 | - | - | - | - | - | - | - |
| Phytol | - | 0,05 | 0,02 | 0,05 | 0,02 | 0,02 | - | - |
| Farnesol | - | - | - | - | - | - | 0,1 | 0,1 |
| Prozentuale Sebumreduktion | 28 | 48 | 33 | 40 | 97 | 83 | 77 | 18 |

## 2. Rezepturbeispiele

### 2.1 Shampoo für fettendes Haar

| | | | |
|---|---|---|---|
| Texapon(R) N 25 | (1) | 40 | Gew.-% |
| Comperlan(R) KD | (2) | 3 | |
| 4-Isononyloxy-benzoesäure | | 0,04 | |
| D,L-alpha-Tocopherol | | 0,2 | |
| Bronidox(R) L | (3) | 0,2 | |
| Wasser | | ad 100 | |

### 2.2 Schnellhaarkur-Emulsion

| | | | |
|---|---|---|---|
| Cetylalkohol | | 3,0 | Gew.-% |
| Dehyquart(R) A | (4) | 2,0 | |
| 4-Isotridecyloxy-benzoesäure | | 0,004 | |
| 2,6-Di-tert.butyl-4-methylphenol | | 0,2 | |
| Citronensäure | | 1,0 | |
| Wasser | | ad 100 | |

### 2.3 Schnellhaarkur, klar

| | | | |
|---|---|---|---|
| Cetiol(R) HE | (5) | 20,0 | Gew.-% |
| Cetylpyridiniumchlorid | | 5,0 | |
| Glycerin | | 5,0 | |
| 4-Dodecyloxy-benzylalkohol | | 0,05 | |
| Octopirox(R) | | 0,1 | |
| Isopropanol | | ad 100 | |

### 2.4 Haarwasser

| | | | |
|---|---|---|---|
| Cetiol(R) HE | (5) | 2,0 | Gew.-% |
| Birkenextrakt | | 1,0 | |
| 4-Isononyloxy-benzoesäure | | 0,02 | |
| Octopirox(R) | | 0,1 | |
| Isopropanol | | 30,0 | |
| Wasser | | ad 100 | |

2.5 Hautemulsion O/W

| | | | |
|---|---|---|---|
| Cutina(R)MD | (6) | 7,0 | Gew.-% |
| Eumulgin(R)B1 | (7) | 3,0 | |
| Cetiol(R)SN | (8) | 10,0 | |
| Myritol(R)318 | (9) | 10,0 | |
| 4-Isononyloxy-benzoesäure | | 0,01 | |
| Phytol | | 0,05 | |
| Wasser | | ad 100 | |

2.6 Hautcreme O/W

| | | | |
|---|---|---|---|
| Cutina(R)MD | (6) | 17 | Gew.-% |
| Eumulgin(R)B1 | (7) | 3 | |
| Eutanol(R)G | (10) | 11 | |
| Myritol(R)318 | (9) | 6 | |
| Karottenöl CLR | | 3 | |
| 4-Isotridecyloxy-benzoesäure | | 0,002 | |
| Farnesol | | 0,02 | |
| Wasser | | ad 100 | |

Die in den Rezepturbeispielen verwendeten Handelsnamen haben folgende Bedeutung:

(1) Texapon(R)N 25: 28%ige wäßrige Lösung von Alkyl-($C_{12}$-$C_{14}$)poly(2 EO)glycolethersulfat-Na-Salz (Henkel KGaA)

(2) Comperlan(R)KD: Kokosfettsäurediethanolamid (Henkel KGaA)

(3) Bronidox(R)L: 5-Brom-5-nitro-1,3-dioxan (10%ige Lösung in 1,2-Propylenglycol) (Henkel KGaA)

(4) Dehyquart(R)A: Cetyltrimethylammoniumchlorid (25%ige Lösung in Wasser) (Henkel KGaA)

(5) Cetiol(R)HE: Polyol-Fettsäureester (CTFA-Bezeichnung: PEG-7-Glyceryl-Cocoate) (Henkel KGaA)

(6) Cutina(R)MD: Palmitin/stearinsäure-mono/diglycerid (Henkel KGaA)

(7) Eumulgin(R) B1: Cetyl/stearylalkohol + 12 Mol Ethylenoxid (Henkel KGaA)

(8) Cetiol(R) SN: Cetyl/stearyl-isononanoat (Henkel KGaA)

(9) Myritol(R) 318: Capryl/caprinsäure-triglycerid (Henkel KGaA)

(10) Eutanol(R) G: 2-Octyldodecanol (Henkel KGaA)

(11) Octopirox(R): 1-Hydroxy-4-methyl-6-(2,4,4-trimethylpentyl)-2-pyridon, Monoethanolammonium-Salz (Hoechst AG)

## Patentansprüche

1. Sebosuppressive Zubereitungen, enthaltend
   (a) eine oder mehrere antiseborrhoisch wirksame Verbindung(en) der Formel (I)

(I)

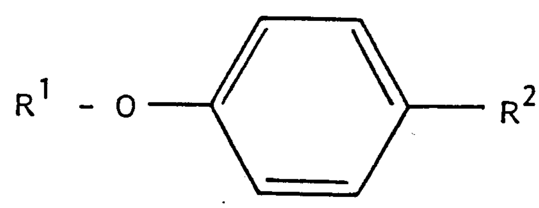

in der $R^1$ eine lineare oder verzweigte, gesättigte oder ungesättigte, aliphatische oder cycloaliphatische Alkylgruppe mit bis zu 20 C-Atomen, eine Hydroxyalkylgruppe mit 2 bis 20 C-Atomen, eine Alkoxyalkylgruppe mit insgesamt 3 bis 20 C-Atomen oder eine Benzylgruppe ist, die am Ring durch ein Halogen, eine Alkyl- oder Alkoxygruppe mit 1 bis 6 C-Atomen substituiert sein kann, und $R^2$ eine Hydroxyalkylgruppe mit 1 bis 4 C-Atomen, eine Gruppe $-COOR^3$, $-CH_2-COOR^3$, $-CH_2-CH_2-COOR^3$ oder $-CH=CH-COOR^3$ ist, worin $R^3$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy- und 2 bis 4 C-Atomen in der Alkylgruppe oder Wasserstoff oder ein salzbildendes Kation ist und
   (b) eine oder mehrere synergistisch wirkende Verbindung(en), ausgewählt aus
   (b1) Antioxidantien, bevorzugt aus der Gruppe der Tocopherole, der tert, butylsubstituierten Phenole und der Gallussäureester
   (b2) antimikrobiellen Wirkstoffen, bevorzugt aus der Gruppe
   1-Hydroxy-2-pyridone und deren Salze
   1-Hydroxy-2-pyridin-thione und deren Salze
   Bis-(2-pyridyl-1-oxid)-disulfid und deren Erdalkalisalz-addukte und
   1-(4-Chlorphenoxy)-1-(1-imidazolyl)-3,3-dimethyl-2-butanon und
   (b3) aliphatischen verzweigten und einfach oder mehrfach ungesättigten Terpenalkoholen mit 8 bis 26 C-Atomen.

2. Sebosuppressive Zubereitungen nach Anspruch 1, dadurch gekennzeichnet, daß in den antiseborrhoisch wirksamen Verbindungen der Formel (I) $R^1$ eine verzweigte, eine cycloaliphatische und/oder einfach oder mehrfach ungesättigte Alkylgruppe mit 8 bis 20 C-Atomen und $R^2$ eine Gruppe $-CH_2OH$, $-COOR^3$, $-CH_2-COOR^3$, $-CH_2-CH_2-COOR^3$ oder $-CH=CH-COOR^3$ ist, worin $R^3$ eine Alkylgruppe mit 1

bis 4 C-Atomen, Wasserstoff oder ein salzbildendes Kation ist.

**3.** Sebosuppressive Zubereitungen nach Anspruch 2, dadurch gekennzeichnet, daß die synergistisch wirksame Verbindung ausgewählt ist aus
(b1) D-alpha-Tocopherol, D,L-alpha-Tocopherol, Tocopherolgemischen, 2,6-Di-tert.butyl-4-methylphenol
(b2) 1-Hydroxy-4-methyl-6-(2,4,4-trimethylpentyl)-2-pyridon oder dessen Salzen
(b3) Phytol und Farnesol.

**4.** Verwendung von Zubereitungen nach den Ansprüchen 1 bis 3 zur Herstellung eines Arzneimittels zur topischen Behandlung seborrhoischer Erkrankungen oder kosmetischer Präparate zur Pflege der Haut und des Haars, dadurch gekennzeichnet, daß man (a) die Verbindungen der Formel (I) und die synergistisch wirkenden Verbindungen (b) in einen dermatologisch verträglichen Träger einarbeitet.

## Claims

**1.** Sebosuppressive preparations containing
(a) one or more antiseborrhoeic compounds corresponding to formula (I)

(I)

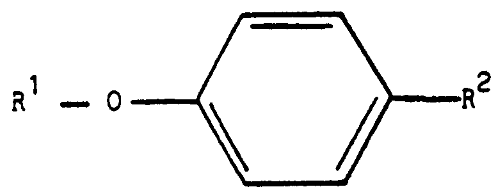

in which $R^1$ is a linear or branched, saturated or unsaturated, aliphatic or cycloaliphatic alkyl group containing up to 20 C atoms, a hydroxyalkyl group containing 2 to 20 C atoms, an alkoxyalkyl group containing a total of 3 to 20 C atoms or a benzyl group, which may be substituted at the ring by a halogen, a $C_{1-6}$ alkyl or alkoxy group, and $R^2$ is a hydroxyalkyl group containing 1 to 4 C atoms, a group $-COOR^3$, $-CH_2-COOR^3$, $-CH_2-CH_2-COOR^3$ or $-CH=CH-COOR^3$, in which $R^3$ is a $C_{1-4}$ alkyl group, a $C_{2-4}$ hydroxyalkyl group, an alkoxyalkyl group containing 1 to 4 C atoms in the alkoxy group and 2 to 4 C atoms in the alkyl group or hydrogen or a salt-forming cation, and
(b) one or more synergistic compounds selected from
(b1) antioxidants, preferably from the group consisting of tocopherols, tert.-butyl-substituted phenols and gallic acid esters,
(b2) antimicrobial agents, peferably from the group consisting of 1-hydroxy-2-pyridones and salts thereof, 1-hydroxy-2-pyridinethiones and salts thereof, bis-(2-pyridyl-1-oxide)-disulfide and alkaline earth metal salt adducts thereof and 1-(4-chlorophenoxy)-1-(1-imidazolyl)-3,3-dimethyl-2-butanone and
(b3) aliphatic, branched and mono- or polyunsaturated $C_{8-26}$ terpene alcohols.

**2.** Sebosuppressive preparations as claimed in claim 1, characterized in that, in the antiseborrhoeic compounds of formula (I), $R^1$ is a branched, cycloaliphatic and/or mono-or polyunsaturated $C_{8-20}$ alkyl group and $R^2$ is a group $-CH_2OH$, $-COOR^3$, $-CH_2-COOR^3$, $-CH_2-CH_2-COOR^3$ or $-CH=CH-COOR^3$, in which $R^3$ is a $C_{1-4}$ alkyl group, hydrogen or a salt-forming cation.

**3.** Sebosuppressive preparations as claimed in claim 2, characterized in that the synergistic compound is selected from
(b1) D-alpha-tocopherol, D,L-alpha-tocopherol, tocopherol mixtures, 2,6-di-tert.butyl-4-methylphenol
(b2) 1-hydroxy-4-methyl-6-(2,4,4-trimethylpentyl)-2-pyridone or salts thereof
(b3) phytol and farnesol.

**4.** The use of the preparations claimed in claims 1 to 3 for the production of a medicament for the topical treatment of seborrhoeic complaints or cosmetic skin-care and hair-care preparations, characterized in that (a) the compounds of formula (I) and the synergistic compounds (b) are incorporated in a

dermatologically safe vehicle.

**Revendications**

**1.** Compositions supprimant la séborrhée contenant :

a) un ou plusieurs composés actifs contre la séborrhée de formule (1)

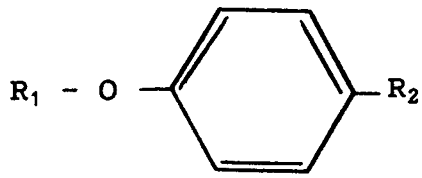

(I)

dans laquelle $R_1$ est un radical alcoyle linéaire ou ramifié, saturé ou non saturé, aliphatique ou cycloaliphatique ayant jusqu'à 20 atomes de carbone, un radical hydroxyalcoyle ayant de 2 à 20 atomes de carbone, un radical alcoxyalcoyle avec au total de 3 à 20 atomes de carbone ou un radical benzyle, qui peut être substitué sur le noyau par un halogène; un radical alcoyle ou un radical alcoxy ayant de 1 à 6 atomes de carbone et $R_2$ est un radical hydroxyalcoyle ayant de 1 à 4 atomes de carbone, un groupe $-COOR_3$, $-CH_2-COOR_3$, $-CH_2-CH_2-COOR_3$ ou $-CH=CH-COOR_3$, dans lesquels $R_3$ est un radical alcoyle ayant de 1 à 4 atomes de carbone, un radical hydroxyalcoyle ayant de 2 à 4 atomes de carbone, un radical alcoxyalcoyle ayant de 1 à 4 atomes de carbone dans le radical alcoxy et de 2 à 4 atomes de carbone dans le radical alcoyle, ou de l'hydrogène ou un cation formant un sel, et

b) un ou plusieurs composé(s) agissant d'une manière synergique choisi(s) parmi

b1) les antioxydants, de préférence dans le groupe des tocophérols, des phénols substitués par un terbutyle et des esters d'acide gallique,

b2) des substances actives antimicrobiennes de préférence dans le groupe de la 1-hydroxy-2-pyridone et ses sels, la 1-hydroxy-2-pyridinethione et ses sels, le disulfure de bis-(2-pyridyl-1-oxyde) et ses composés d'addition sur les sels de métaux alcalino-terreux et le 1-(4-chlorophénoxy)-1-1-imidazolyl)-3,3-diméthyl-2-butanone et

b3) des alcools terpéniques aliphatiques ramifiés et une fois ou plusieurs fois insaturés, ayant de 8 à 26 atomes de carbone.

**2.** Compositions supprimant la séborrhée selon la revendication 1, caractérisées en ce que dans les composés actifs contre la séborrhée de formule (I), $R_1$ est un radical alcoyle ramifié, un radical cycloaliphatique et/ou un radical alcoyle une fois ou plusieurs fois insaturé ayant de 8 à 20 atomes de carbone et $R_2$ est un groupe $-CH_2OH$, $-COOR_3$, $-CH_2-COOR_3$, $-CH_2-CH_2-COOR_3$ ou $-CH=CH-COOR_3$ dans lesquels $R_3$ est un radical alcoyle ayant de 1 à 4 atomes de carbone, de l'hydrogène ou un cation formant un sel.

**3.** Compositions supprimant la séborrhée selon la revendication 2, caractérisées en ce que le composé actif d'une manière synergique est choisi parmi :

b1) le D-$\alpha$-tocophérol, le D,L-$\alpha$-tocophérol, les mélanges de tocophérols, le 2,6-di-terbutyl-4-méthylphénol,

b2) la 1-hydroxy-4-méthyl 6-(2,4,4-triméthyl pentyl)- 2-pyridone ou ses sels

b3) le phytol et le farnesol.

**4.** Utilisation des compositions selon les revendications 1 à 3, pour l'obtention d'un médicament pour le traitement topique des maladies séborrhéiques ou de préparations cosmétiques pour les soins de la peau et des cheveux, caractérisée en ce que l'on incorpore a) les composés de formule 1 et les composés qui agissent d'une manière synergique b) dans un support compatible dermatologiquement.